# EUROPEAN PATENT APPLICATION

(11) **EP 4 451 281 A1**
(43) Date of publication of application: **23.10.2024**
(21) Application number: 23168413.5
(22) Date of filing: 18.04.2023
(51) Int. Cl.: G16H 10/60, G06F 40/00, G06N 3/00, G06N 20/00, G16H 15/00

(54) **SYSTEM AND METHOD FOR ANONYMIZING MEDICAL RECORDS**

(71) Applicant: Claritrics Inc d.b.a Buddi AI, New York, NY 10036 (US)
(72) Inventor: RAJKUMAR, Sriram, 600024 Chennai (IN); SANTHIAPPAN, Sudarsun, 600077 Chennai (IN)
(74) Representative: Pizzoli, Antonio Mario

(57) **Abstract**

The present disclosure describes a method, apparatus, and computer readable medium for anonymizing medical records using a combination of deep learning and smart templatization. The method comprises performing tokenization on an input medical record comprising one or more sentences to generate tokenized data and generating one or more templatized sentences by performing templatization on the tokenized data, where performing the templatization comprises replacing one or more known patterns in the tokenized data with predefined patterns. The method further comprises identifying one or more PHI sentences from the templatized sentences using a trained classifier, each PHI sentence may comprise one or more PHI. The method further comprises identifying the PHI in the medical record by processing the identified PHI sentences using a trained model and generating an anonymized medical record by anonymizing the identified PHI in the input medical record.

## Description

### TECHNICAL FIELD

The present disclosure generally relates to the technical field of natural language processing and machine learning. Particularly, the present disclosure relates to a system and a method for anonymizing selective information present in medical records.

### BACKGROUND

A medical record (MR) is a systematic documentation comprising information related to health of a patient and personal data of the patient to aid in diagnosis and treatment of the patient. The MR may also be used for various allied services such as, but not limited to, medical/ life-science research (i.e., educating medical students and physicians), studying healthcare trends, data mining, planning patient care, insurance claims, improving clinical care etc. For these allied services the MR may need to be shared with outside entities (i.e., entities which are outside of a health care facility). The outside entities may include institutions, organizations, or persons.

MR comprises sensitive information related to patients (i.e., protected health information (PHI)). PHI stands for Protected Health Information, also referred to as Personal Health Information, and may include any information present in the MR which can be used to identify an individual or patient. In the United States (US), Health Insurance Portability and Accountability Act (HIPAA) is a regulation which governs secure handling of the PHI. HIPAA governs how health care facilities and others can use and share the PHI. Since MR comprises sensitive information (i.e., PHI), it cannot be shared directly with the outside entities due to privacy constraints. Hence, MR should be shared with the outside entities only after proper preconditioning i.e., after removing or replacing the PHI from the MR. One way of preconditioning the MR is its de-identification i.e., removal or replacement of all PHI contained in the MR.

De-identification of the medical records is a time consuming and challenging task. Traditional techniques of medical record deidentification have low performance and mainly rely on structured/semi-structured medical records to precisely identify PHI. Thus, medical record deidentification is still regarded as a complex problem and it is desirable to develop efficient techniques for medical record deidentification which can accurately identify the PHI present in medical records. Hence, there exists a need for further improvements in the technology, especially for techniques that can accurately identify PHI present in the medical records.

The information disclosed in this background section is only for enhancement of understanding of the general background of the invention and should not be taken as an acknowledgement or any form of suggestion that this information forms the prior art already known to a person skilled in the art.

### SUMMARY

One or more shortcomings discussed above are overcome, and additional advantages are provided by the present disclosure. Additional features and advantages are realized through the techniques of the present disclosure. Other embodiments and aspects of the disclosure are described in detail herein and are considered a part of the disclosure.

An object of the present disclosure is to de-identify and re-identify PHI entities in a medical record using a combination of rules, deep learning, and smart templatization.

Another objective of the present disclosure is to provide medical record deidentification techniques which can handle medical records of any type (i.e., structured, unstructured, semi-structured medical records).

Another object of the present disclosure is to accurately anonymize medical records in a time and resource efficient manner.

The above stated objects as well as other objects, features, and advantages of the present disclosure will become clear to those skilled in the art upon review of the following description, the attached drawings, and the appended claims.

According to an aspect of the present disclosure, methods, apparatus, and computer readable media are provided for anonymizing medical records.

In a non-limiting embodiment of the present disclosure, the present application discloses a method for anonymizing medical records. The method may comprise performing tokenization on an input medical record comprising one or more sentences to generate tokenized data, where the tokenized data comprises one or more tokenized sentences. The method may further comprise generating one or more templatized sentences by performing templatization on the tokenized data, where performing the templatization comprises replacing one or more known patterns in the tokenized data with one or more predefined patterns. The method may further comprise identifying one or more Protected Health Information (PHI) sentences from the templatized sentences by processing the templatized sentences using a trained classifier, wherein each of the one or more PHI sentences comprises one or more PHI. The method may further comprise identifying one or more PHI in the medical record by processing the identified PHI sentences using a trained model and generating an anonymized medical record by anonymizing the identified PHI in the input medical record. The method may further comprise transmitting the anonymized medical record to an external entity.

In another non-limiting embodiment of the present disclosure, the present application discloses an apparatus for anonymizing medical records. The apparatus may comprise a memory storing computer executable instructions and at least one processor in electronic communication with the memory. The at least one processor may be configured to perform tokenization on an input medical record comprising one or more sentences to generate tokenized data, where the tokenized data comprises one or more tokenized sentences. The at least one processor may be further configured to generate one or more templatized sentences by performing templatization on the tokenized data, where performing the templatization comprises replacing one or more known patterns in the tokenized data with one or more predefined patterns. The at least one processor may be further configured to identify one or more Protected Health Information (PHI) sentences from the templatized sentences by processing the templatized sentences using a trained classifier, wherein each of the one or more PHI sentences comprises one or more PHI. The at least one processor may be further configured to identify one or more PHI in the medical record by processing the identified PHI sentences using a trained model. The at least one processor may be further configured to generate an anonymized medical record by anonymizing the identified PHI in the input medical record and transmit the anonymized medical record to an external entity.

In another non-limiting embodiment of the present disclosure, the present application discloses a non-transitory computer readable media storing one or more instructions executable by at least one processor. The one or more instructions may comprise one or more instructions for performing tokenization on an input medical record comprising one or more sentences to generate tokenized data, where the tokenized data comprises one or more tokenized sentences. The one or more instructions may further comprise one or more instructions for generating one or more templatized sentences by performing templatization on the tokenized data, where performing the templatization comprises replacing one or more known patterns in the tokenized data with one or more predefined patterns. The one or more instructions may further comprise one or more instructions for identifying one or more Protected Health Information (PHI) sentences from the templatized sentences by processing the templatized sentences using a trained classifier, where each of the one or more PHI sentences comprises one or more PHI. The one or more instructions may further comprise one or more instructions for identifying one or more PHI in the medical record by processing the identified PHI sentences using a trained model. The one or more instructions may further comprise one or more instructions for generating an anonymized medical record by anonymizing the identified PHI in the input medical record and one or more instructions for transmitting the anonymized medical record to an external entity.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF DRAWINGS

Further aspects and advantages of the present disclosure will be readily understood from the following detailed description with reference to the accompanying drawings. Reference numerals have been used to refer to identical or functionally similar elements. The figures together with a detailed description below, are incorporated in and form part of the specification, and serve to further illustrate the embodiments and explain various principles and advantages, in accordance with the present disclosure wherein:
**Figure 1** shows an exemplary communication system **100** for use in deidentifying/anonymizing medical records, in accordance with some embodiments of the present disclosure.
**Figure 2** shows a block diagram **200** of the communication system **100** illustrated in **Figure 1****,** in accordance with some embodiments of the present disclosure.
**Figure 3** shows a process flow diagram **300** for anonymizing medical records, in accordance with some embodiments of the present disclosure.
**Figure 4(a)** shows a detailed representation **400-1** of a PHI sentence classifier, in accordance with some embodiments of the present disclosure.
**Figure 4(b)** shows a detailed representation **400-2** of a deep-learning based LSTM-CRF classifier, in accordance with some embodiments of the present disclosure.
**Figure 4(c)** shows a detailed representation **400-3** of the deep-learning based model for identifying PHI in the identified PHI sentences, in accordance with some embodiments of the present disclosure.
**Figure 5** shows a detailed block diagram **500** of the computing system **110, 120** for anonymizing medical records, in accordance with some embodiments of the present disclosure.
**Figure 6** depicts a flowchart **600** illustrating a method for anonymizing medical records, in accordance with some embodiments of the present disclosure.

It should be appreciated by those skilled in the art that any block diagrams herein represent conceptual views of the illustrative systems embodying the principles of the present disclosure. Similarly, it will be appreciated that any flowcharts, flow diagrams, state transition diagrams, pseudo code, and the like represent various processes which may be substantially represented in computer readable medium and executed by a computer or processor, whether or not such computer or processor is explicitly shown.

### DETAILED DESCRIPTION

In the present document, the word "exemplary" is used herein to mean "serving as an example, instance, or illustration." Any embodiment or implementation of the present disclosure described herein as "exemplary" is not necessarily to be construed as preferred or advantageous over other embodiments.

While the disclosure is susceptible to various modifications and alternative forms, specific embodiments thereof have been shown by way of example in the drawings and will be described in detail below. It should be understood, however, that it is not intended to limit the disclosure to the particular form disclosed, but on the contrary, the disclosure is to cover all modifications, equivalents, and alternatives falling within the spirit and the scope of the disclosure.

The terms "comprise(s)", "comprising", "include(s)", or any other variations thereof, are intended to cover a non-exclusive inclusion, such that a setup, device, apparatus, system, or method that comprises a list of components or steps does not include only those components or steps but may include other components or steps not expressly listed or inherent to such setup or device or apparatus or system or method. In other words, one or more elements in a device or system or apparatus proceeded by "comprises... a" does not, without more constraints, preclude the existence of other elements or additional elements in the system.

In the following detailed description of the embodiments of the disclosure, reference is made to the accompanying drawings that form a part hereof, and in which are shown by way of illustration of specific embodiments in which the disclosure may be practiced. These embodiments are described in sufficient detail to enable those skilled in the art to practice the disclosure, and it is to be understood that other embodiments may be utilized and that changes may be made without departing from the scope of the present disclosure. The following description is, therefore, not to be taken in a limiting sense. In the following description, well known functions or constructions are not described in detail since they would obscure the description with unnecessary detail.

The terms like "at least one" and "one or more" may be used interchangeably throughout the description. The terms like "a plurality of' and "multiple" may be used interchangeably throughout the description. Further, the terms like "deidentification" and "anonymization" may be used interchangeably throughout the description. The terms like "medical record (MR)", "health record (HR)", "electronic medical record (EMR)", and "electronic health record (EHR)" may be used interchangeably throughout the description.

In the present disclosure, the term "medical record" is used within the context of its broadest definition. A medical record (MR) is an integral part of healthcare system and may refer to a documentary evidence comprising information related to various healthcare events in the life of a person/patient (i.e., patient's health history and past medical examination reports) as well as identification of the patient (i.e., personal data of the patient). MR aids in diagnosis and treatment of the patient and may also be used for various allied services. MR can be in the form of a paper record, or an electronic/digital record, or a combination of both. An electronic health record (EHR) or electronic medical record (EMR) is a real-time record that makes secure and instant availability of health information of a person. The medical record may comprise both structured and unstructured data and could be in any form including, but not limited to, text, images, word files, web pages, excel, PDFs etc.

In the present disclosure, the term "Protected Health Information (PHI)" is used within the context of its broadest definition. PHI is information, including demographic information, which relates to: an individual's past, present, or future physical or mental health or condition; provision of health care to the individual; or past, present, or future payment for the provision of health care to the individual, and that identifies the individual or for which there is a reasonable basis to believe can be used to identify the individual. Some information that can be considered PHI are names, surnames, addresses, birth dates, Social Security Numbers, phone numbers, fax numbers, email addresses, medical record numbers, account numbers, vehicle identifiers, web Uniform Resource Locators (URLs), Internet Protocol (IP) address numbers, billing information etc. present in the medical records. In short, PHI may include any information present in the medical records which, either alone or along with other information, may be used to identify an individual or a patient.

In the present disclosure, the term "de-identification" is used within the context of its broadest definition. In general, de-identification is the process of preventing someone's personal identity from being revealed. De-identification removes personal identifiers, both direct and indirect, that may lead to an individual being identified. In the present disclosure, removing or replacing the PHI from the MR may be referred as de-identification. An ideal de-identified medical record should be free from all information that can be used to identify an individual.

As discussed in the background section, deidentification of medical records is a challenging task. Medical record deidentification could be carried out by a qualified expert. However, the qualified experts have to manually process a large number of medical records which is time consuming, expensive, and ineffective. Another approach of deidentifying a medical record is removal of certain identifiers or PHI from the medical record using computer based techniques which may include traditional Natural Language Processing (NLP) techniques and Artificial intelligence (AI) based techniques.

For deidentifying the PHI in the medical record, first problem is to identify the PHI in the medical record. The process of identifying the PHI falls under well-known problem of identifying named entities called Named Entity Recognition (NER). In data mining, a named entity is a phrase that clearly identifies one item among a set of other similar items. Examples of named entities are names, geographic locations, addresses, phone numbers etc. NER is a Natural Language Processing (NLP) technique that automatically identifies named entities in a text and classifies them into predefined categories. NER can be tackled by traditional NLP or by machine learning. Traditional NLP based systems utilize custom built rules and dictionary-based methods to identify PHI. However, these systems have low performance because dictionary size is not limited and keeps on increasing. Rule-based approaches can precisely identify PHI in medical records but it is impossible to derive all possible rules for any system to identify PHI. Moreover, since medical records may have different formats and languages, the rule-based techniques require constant updating of rules, which is troublesome and time consuming.

NER has also been tackled using machine learning techniques. However, the machine learning based techniques require task-specific or PHI related features to identify PHI in the medical record. Thus, Deep Learning (DL) based techniques have been used for MR deidentification. The DL based approaches have the advantage over machine learning methods as they don't require task-specific features to identify PHI words. DL can learn these features directly from the input medical records using the context and the output PHI. The problem with DL based approaches is the DL based approaches are not able to attain a high recall and precision which is required for PHI deidentification. The leakage of PHI is a criminal offense and masking non-PHI words may lead to loss of information for downstream tasks. Moreover, the complexity of trained DL model increases with increase in dictionary of words used while training. The dictionary size is correlated with number of unique training records.

One major challenge associated with medical record deidentification is the heterogeneity of data present in the medical records. Modern day medical records usually comprise unstructured data present in different formats that is usually not so easily searchable. The conventional PHI de-identification systems mainly rely on structured/semi-structured documents to precisely identify PHI in medical records. The conventional PHI de-identification systems usually focus on a single type of data and are unable to deidentify unstructured medical records in an efficient manner.

Due to the above-mentioned challenges, medical record deidentification is still regarded as a complex problem and it is desirable to develop an effective and efficient medical record deidentification system which can handle medical records of any type.

To overcome these and other problems, the present disclosure proposes techniques for anonymizing medical records using a combination of rules, deep learning, and smart templatization.

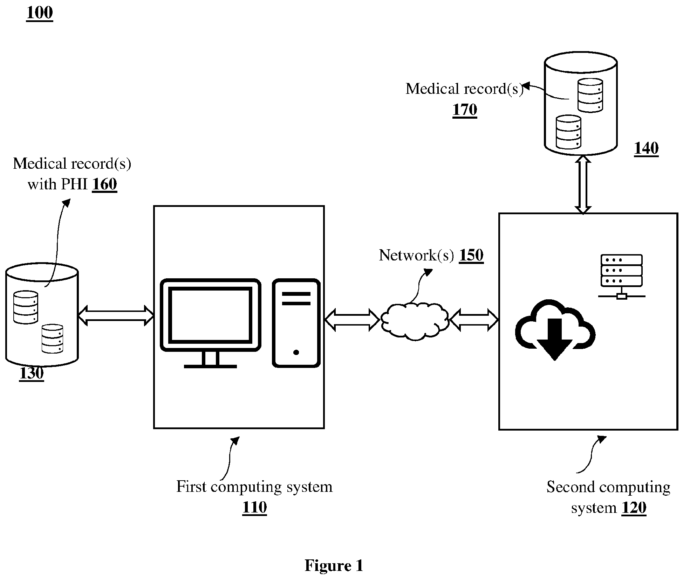

Referring now to **Figure 1****,** which illustrates a communication system **100** for use in deidentifying/anonymizing medical records, in accordance with some embodiments of the present disclosure. The communication system **100** may comprise of a first computing system **110** (or client computing system) which may be in communication with one or more first data sources **130.** The one or more first data sources **130** may comprise at least one medical record **160** (having PHI therein) which is to be deidentified. The communication system **100** may further comprise a second computing system **120** (or a server) in communication with the first computing system **110** via at least one network **150.** Additionally, the second computing system **120** may be in communication with one or more second data sources **140.** The one or more second data sources **140** may comprise at least one medical record **170** for training the second computing system **120.**

The network **150** may comprise a data network such as, but not restricted to, the Internet, Local Area Network (LAN), Wide Area Network (WAN), Metropolitan Area Network (MAN), etc. In certain embodiments, the network **150** may include a wireless network, such as, but not restricted to, a cellular network and may employ various technologies including Enhanced Data rates for Global Evolution (EDGE), General Packet Radio Service (GPRS), Global System for Mobile Communications (GSM), Internet protocol Multimedia Subsystem (IMS), Universal Mobile Telecommunications System (UMTS) etc. In one embodiment, the network **150** may include or otherwise cover networks or subnetworks, each of which may include, for example, a wired or wireless data pathway.

The first and second data sources **130, 140** may be any data source comprising huge volumes of data and/or information (medical records). The first and second data sources **130, 140** may include paper and/or computer based medical records including electronic medical records, lab reports, patient's clinical records, patient's medical history and medication records etc. The first computing system **110** may fetch/receive the at least one medical record **160** from the at least one first data source **130** and the second computing system **120** may fetch/receive at least one medical record **170** from the at least one second data source **140.**

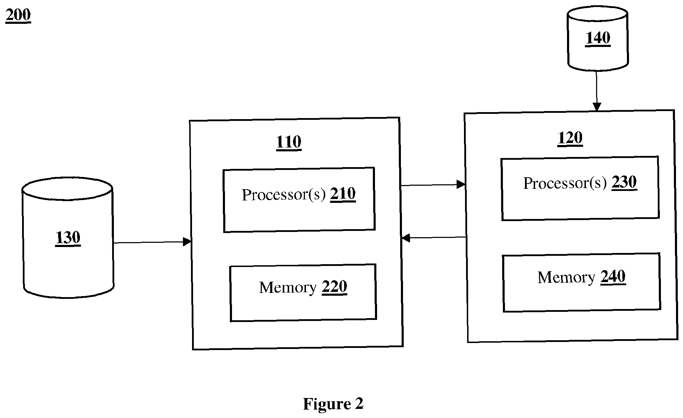

Now, **Figure 1** is explained in conjunction with **Figure 2****,** which is a block diagram 200 of the communication system **100,** in accordance with some embodiments of the present disclosure. According to an embodiment of the present disclosure, the communication system **100, 200** may comprise the first computing system **110,** the second computing system **120,** the at least one first data source **130,** and the at least one second data source **140.** The first computing system **110** may comprise at least one first processor **210,** at least one first memory **220,** and at least one first transceiver (not shown). Similarly, the second computing system **120** may comprise at least one second processor **230,** at least one second memory **240,** and at least one second transceiver (not shown).

The first and second processors **210, 230** may include, but not restricted to, a general-purpose processor, a Field Programmable Gate Array (FPGA), an Application Specific Integrated Circuit (ASIC), a Digital Signal Processor (DSP), microprocessors, microcomputers, micro-controllers, central processing units, state machines, logic circuitries, and/or any devices that manipulate signals based on operational instructions. A processor may also be implemented as a combination of computing devices, e.g., a combination of a DSP and a microprocessor, a plurality of microprocessors, one or more microprocessors in conjunction with a DSP core, or any other such configuration.

The first memory **220** may be communicatively coupled to the at least one first processor **210** and the second memory **240** may be communicatively coupled to the at least one second processor **230.** The first and second memory **220, 240** may comprise various instructions, one or more datasets, and one or more medical records etc. The first and second memory **220, 240** may include a Random-Access Memory (RAM) unit and/or a non-volatile memory unit such as a Read Only Memory (ROM), optical disc drive, magnetic disc drive, flash memory, Electrically Erasable Read Only Memory (EEPROM), a memory space on a server or cloud and so forth.

The communication system **100** proposed in the present disclosure may be named as a medical record processing system which may perform deidentification on a given medical record. The medical record processing system may also perform reidentification on a deidentified medical record. In the forthcoming paragraphs it is considered that the first computing system (i.e., the client device) **110** provides a medical record **160** to the second computing system **120** (i.e., the server) and the processing (deidentification/reidentification) of the medical record **160** is performed at the second computing system **120.** However, the present disclosure is not limited thereto and the processing (deidentification/ reidentification) of the medical record **160** may be performed at the first computing system **110** as well (i.e., at client device). In one embodiment, the first computing system **110** may be located at customer premises and the second computing system **120** may be remotely located. In another embodiment, both the first and second computing systems **110, 120** may be located at the customer premises.

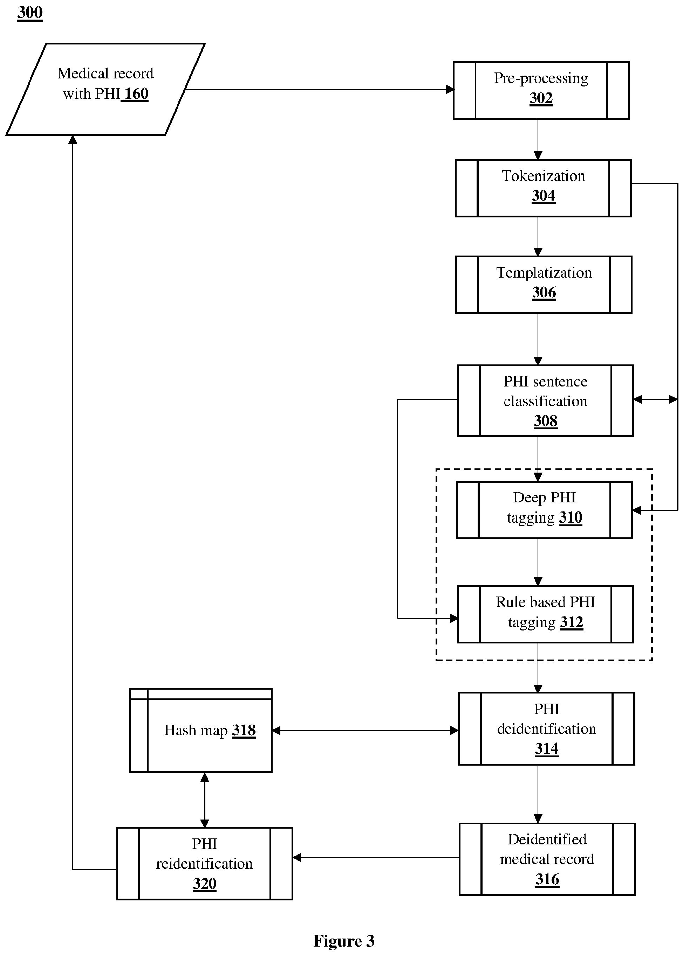

In one non-limiting embodiment of the present disclosure, the at least one first processor **210** may fetch/extract at least one medical record **160** (which is having PHI and which is to be deidentified) from the at least one first data source **130.** In one non-limiting embodiment, the medical record **160** may be provided/transmitted to the first processor **210.** The at least one first processor **210** may transmit the medical record **160** to the at least one second processor **230** of the second computing system **120.** The at least one second processor **230** may process the received medical record **160** for replacing/removing the PHI contained in the medica record **160.** The at least one second processor **230** may use a combination of rules, deep learning, and smart templatization for medical record deidentification. The processing at the at least one second processor **230** is described below with the help of a process flow diagram **300** as described in **Figure 3****.**

The second computing system **120** may work in two phases: first phase being a training phase and a second phase being an implementation phase. It may be worth noting here that one or more deep learning based models/classifiers are first trained and the deidentification is performed thereafter. The outcome of the training phase may be trained models and/or classifiers. The training phase has not been explained in details in the present disclosure and it is assumed that a person skilled in the art may carry out the training of models/classifiers using the conventional training methods.

Referring now to **Figure 3****,** which illustrates a process flow diagram **300** for medical record deidentification/reidentification, in accordance with some embodiments of the present disclosure. The at least one second processor **230** may receive or fetch the medical record **160** from the first computing system **110.** The input medical record **160** may comprise one or more sentences. Once the input medical record **160** is received at the second computing system **120,** the at least one second processor **230** may perform pre-processing on the received/input medical record **160.** The input medical record may comprise unnecessary data which may not be feasible for deidentification or which adds minimal or no value. Before such medical record can be passed for deidentification, the medical record may need some clean-up or pre-processing (block **302**). The cleaning up may include performing one or more operations comprising: removing stop words, removing special characters, removing punctuations, stemming, lemmatization, removing extra white spaces, and converting the medical record into lowercase or uppercase letters etc., but not limited thereto. The pre-processing may improve accuracy of the deidentification techniques and save computing resources by removing the unnecessary data. For image or PDF input medical record, the pre-processing may involve Optical Character Recognition (OCR) to extract textual content from the input medical record.

In one non-limiting embodiment, pre-processing the input medical record may comprise converting the input medical record (which may be in any format including, but not limited to, text, images, word files, web pages, excel, PDFs etc.) into a defined format (e.g., image or pdf).

In one non-limiting embodiment, pre-processing the input medical record may also comprise merging a sentence of the input medical record with previous or next sentence(s) using a deep learning based context merger classifier. Sometimes line breaks in a medical record may separate a word from its context but the context is important for determining whether the word is PHI or not. Thus, for systems that operate at a single line level, it becomes difficult to determine whether the word is PHI or not as the context is missing at single line level. To fix this issues, the present disclosure uses a context merger classifier for merging a sentence of the input medical record with previous or next sentence(s). The context merger classifier is be a deep learning based model that can understand sequential information in texts and generate vector representation corresponding to the sentences. These vector representations may then be given as input to the trained context merger classifier for merging sentences in the medical record with previous/next sentences, as shown below:

| **Original Input** | **Context merger classifier output** |
|---|---|
| Name: | Name: Jane Doe |
| Jane Doe | |
| Jane Doe has electronically signed this document. | Jane Doe has electronically signed this document. |

In one non-limiting embodiment of the present disclosure, the at least one second processor **230** may perform tokenization on the pre-processed input medical record to generate tokenized data (block **304**). Particularly, the at least one second processor **230** may perform tokenization on the pre-processed input medical record to generate one or more tokenized sentences (i.e., sentences comprising token(s)) corresponding to the one or more sentences of the medical record **160.** Most of the deep learning models generally cannot work with raw medical records. In order to make these models understand data present in medical records, it is required to break that data down into tokens, as shown below:
**Input:** *"Dr. Jane Doe recommended Drug A to Mr. Alex on 20*/*01*/*22"*
**Tokenized output:** *'Dr.' 'Jane' 'Doe' 'recommended' 'Drug A' 'to' 'Mr.'* '*Alex*' *'on' '20'* '/' *'01'* '/' *'22'*

Tokenization is the process of breaking raw data present in a document into a set of meaningful pieces called tokens. The tokens may be either words, or characters, or symbols, or sub-words. The tokens may then be used to prepare a dictionary. The dictionary may refer to a set of unique tokens present in the medical record. It may be noted that the dictionary can be prepared by considering each unique token in the medical record. The dictionary (i.e., the occurrences of tokens in the medical record) can be represented in the form of a vector which in turn converts an unstructured document into a structured numerical data suitable for machine/deep learning models.

The dictionary generated from the medical record may be too large comprising a lot of words thereby, lowering the performance of deep/machine learning models. Hence, it is desirable to reduce the dictionary size by removing the unnecessary words. Though pre-processing helps in reducing the dictionary size but even the pre-processed medical record may comprise a lot of unimportant words/tokens which may not be relevant for the deep/machine learning models.

In one non-limiting embodiment of the present disclosure, the at least one second processor **230** may perform templatization in order to reduce the dictionary size (block **306**). The at least one second processor **230** may generate one or more templatized sentences by performing templatization on the tokenized data. Templatization is the process of replacing one or more known patterns in a sentence with one or more predefined patterns upon satisfying certain conditions or rules. Templatization reduces the number of unique tokens by replacing unimportant patterns with known patterns and hence reduces the dictionary size of the deep/machine learning model, thereby reducing the complexity of the model. In addition to reducing the complexity of the model and making it more efficient, templatization may also help in save computing resources of the computing system. For instance, by reducing the size of the dictionary used by the model, the templatization helps in reducing the memory and processing power required to run the model. This can be particularly important for devices with limited resources, where minimizing computational requirements is essential for optimal performance.
**Input:** *`Dr.' 'Jane' 'Doe' 'recommended' 'Drug A' 'to' 'Mr.'* '*Alex*' *'on' '20'* '/' *'01'* '/' '22'
**Templatized output:** *`Dr.' 'Jane' 'Doe' 'recommended' 'Drug A' 'to' 'Mr.'* '*Alex*' *'on' 'Num_2*' '/' '*Num_2*' '/' '*Num_2*'

In the input sentence, three unique less important patterns (i.e., 20, 01, 22) have been replaced with a single pattern (i.e., Num_2). This way number of unique patterns/words gets reduced thereby reducing the dictionary size. Similarly, if different words/patterns having same meaning are occurring in the medical document, they can be replaced with a single word/pattern. E.g., the words 'male', 'female', 'man', 'woman', 'men', 'women', 'lady', 'gentleman', 'guy', 'boy', 'girl' etc. occurring in the medical record can be replaced with a single pattern/word (e.g., 'gender') in order to reduce the dictionary size of the model. The templates are created by extensive analysis of various patterns encountered in medical records. A few of the templates that may be utilized are numbers, alphanumeric, characters etc. However, the present disclosure is not limited thereto and a plurality of different templates may be created.

In one non-limiting embodiment, apart from reducing the dictionary size, the templatization may act as a signal for a potential PHI following/preceding the template token. For instance, the templatization may create a general signal for potential PHI and this general signal along with a deep learning model can enable better and faster PHI identification. This can be better understood by way of following example:

Consider a templatization condition which can replace the words 'printed'/ 'print'/ 'prints' in a medical record with "Name_1" which means there is a high probability of finding a PHI string following the pattern "Name_1". While processing the medical record using any deep learning model (in block **310**), the at least one second processor **230** upon detecting the word/pattern "Name_1" in the medical record can determine that there are high chances of finding a PHI string following the pattern "Name_1".

| **Input sentences** | **Templatized sentences** |
|---|---|
| 'Printed' 'by': 'Dr.' 'Jane' 'Doe' | **'Name_1'** 'by': 'Dr.' 'Jane' 'Doe' |
| 'Printed' - 'Jane' 'Doe' | **'Name_1'** - 'Jane' 'Doe' |
| 'This' 'medical' 'record' 'was' 'printed' 'at' 'Helix' 'Printing' 'Press' . | 'This' 'medical' 'record' 'was' '**Name_1**' 'at' 'Helix' 'Printing' 'Press'. |
| 'Medical' 'record' 'printed' `by' 'Jane' 'Doe' 'at' 'station' '1043'. | 'Medical' 'record' **`Name_1'** 'by' 'Jane' 'Doe' 'at' 'station' '1043'. |

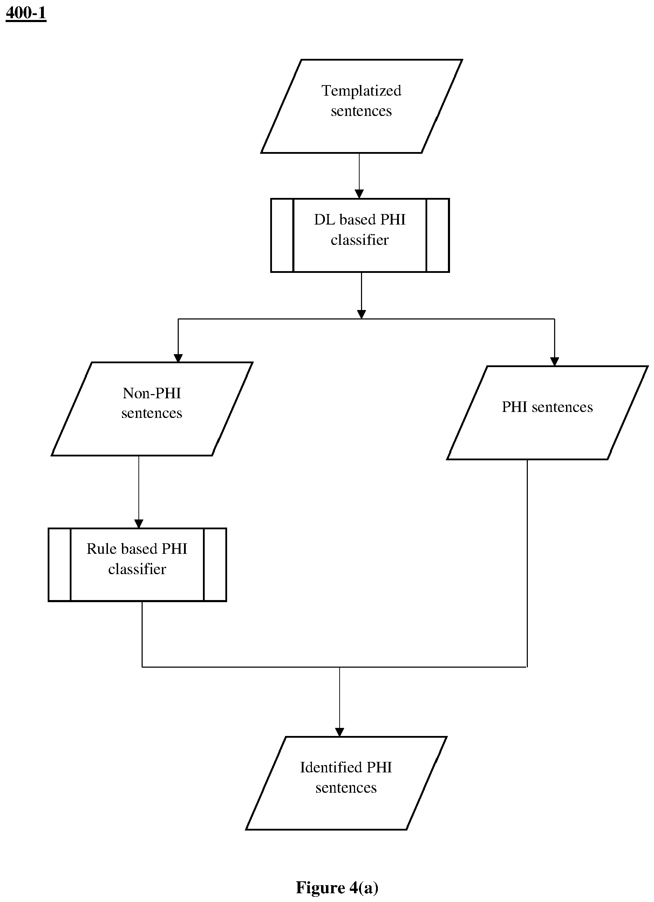

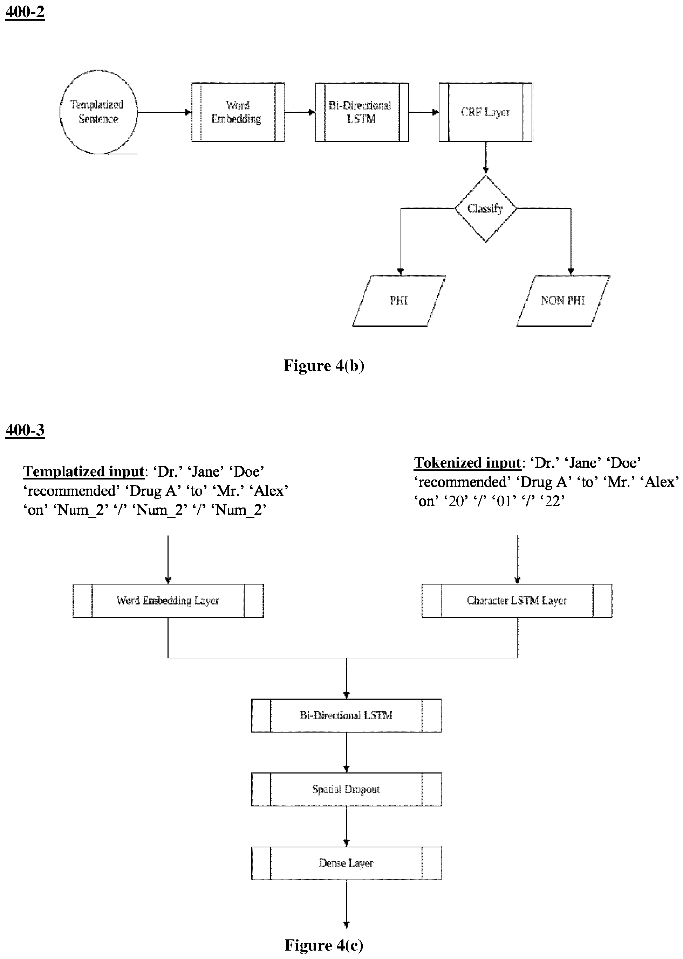

In one non-limiting embodiment of the present disclosure, the at least one second processor **230** may perform PHI sentence classification on the templatized sentences (block **308**). The at least one second processor **230** may classify each of the templatized sentences as a PHI sentence (i.e., sentence comprising at least one PHI) or non-PHI sentence using a PHI sentence classifier, as illustrated further in **Figures 4(a)** and **4(b)****.**

Referring now to **Figure 4(a)** which illustrates a detailed representation **400-1** of the PHI sentence classifier, in accordance with some embodiments of the present disclosure. For performing the PHI sentence classification, the templatized sentences are fed into the PHI sentence classifier that identifies PHI and non-PHI sentences present in the medical record. The PHI sentence classifier is a combination of a deep-learning based classifier and a rule-based classifier. Initially, the at least one second processor **230** may process the templatized sentences using a trained deep-learning based classifier, which classifies each of the templatized sentences as a PHI sentence or a non-PHI sentence. The trained deep-learning based classifier is a sequential deep learning classifier. In an exemplary embodiment, the deep-learning based classifier is LSTM-CRF classifier, as illustrated in **Figure 4(b)****.** However, the present disclosure is not limited thereto and any deep learning model that could understand sequential information could be used in place LSTM-CRF including, but not limited to, Bi-LSTM, RNN, GRU, CRF, Transformer models (BERT), models with attention, temporal CNN or any combination thereof.

Referring now to **Figure 4(b)** which illustrates a detailed representation **400-2** of the deep-learning based LSTM-CRF classifier, in accordance with some embodiments of the present disclosure. LSTM stands for Long Short-Term Memory networks, used in the field of deep learning. LSTM is a variety of recurrent neural networks (RNNs) that are capable of learning long-term dependencies, especially in sequence prediction problems. LSTM has feedback connections, i.e., it is capable of processing the entire sequence of data, apart from single data points such as images. A sequence processing model that consists of two LSTMs: one taking the input in a forward direction and the other in a backwards direction is known as Bidirectional LSTM or Bi-LSTM. Thus, Bi-LSTM are recurrent neural networks formed by just putting two independent LSTMs together in which the networks have both backward and forward information about the sequence at every input. CRF stands for Conditional Random Field which is a discriminative model used for predicting sequences. CRF uses the neighbor tag information for predicting a current tag. Particularly, CRF efficiently uses past and future tags to predict the current tag, which is similar to the use of past and future input features via a bidirectional LSTM network.

In one non-limiting embodiment of the present disclosure, the LSTM-CRF classifier illustrated in **Figure 4(b)** works at sentence level to identify PHI sentences. From the templatized sentence individual words are used to obtain a vector representation of that word also referred as word embedding. The vector representation is learned from a plurality of medical records **170** during training phase. This representation captures the context of the sentences in which the word has occurred during training. The representations are passed to a Bi-LSTM network where another representation is obtained which also depends on the context of the current sentence. Then CRF is used to further refine the representations in order to improve final accuracy. Finally, a dense layer that uses the CRF output predicts the type of sentence (i.e., whether the sentence is a PHI sentence or a non-PHI sentence).

Referring back to **Figure 4(a)****,** the templatized sentences which are classified as non-PHI by the deep learning PHI classifier are again processed using the rule based PHI classifier to identify one or more missed out PHI sentences. A rule-based classifier is another type of classifier which classifies the data using various decisional rules or "if...else" rules. The rules are easily interpretable by the classifier and thus the rule based classifier provides accurate sentence classification. The rule based classifier reinforces the overall recall of PHI sentence classifier. The output of the PHI sentence classifier is a list of sentences classified as PHI and non-PHI from which the sentences which are identified as containing PHI are used for further processing.

Referring back to **Figure 3****,** in one non-limiting embodiment of the present disclosure, the at least one second processor **230** may process the identified PHI sentences using a trained deep learning based model in order to identify one or more PHI contained in the PHI sentences (**block 310-** deep PHI tagging). The detailed representation of the deep learning based model is illustrated in Figure 4(c).

Referring now to **Figure 4(c)****,** which shows a detailed representation **400-3** of the deep learning based model for identifying PHI in the identified PHI sentences, in accordance with some embodiments of the present disclosure. The trained deep learning based model may comprise a plurality of layers comprising a word embedding layer, a character embedding layer, a sequential representation layer, a spatial dropout layer, a dense layer etc. One input to the model is templatized sentences which are classified as PHI (e.g., `Dr.' 'Jane' 'Doe' 'recommended' 'Drug A' 'to' 'Mr.' 'Alex' 'on' 'Num_2' '/' 'Num_2' '/' 'Num_2'). Another input to the model is character based input of tokenized sentences corresponding to the templatized PHI sentences i.e., the second input to model is tokenized PHI sentences and not all tokenized sentences (e.g., `Dr.' 'Jane' 'Doe' 'recommended' 'Drug A' 'to' 'Mr.' 'Alex' 'on' '20' '/' '01' `/' '22'). For instance, the at least one second processor **230** may use a one-to-one mapping between the templatized PHI sentences and the tokenized sentences for selecting the tokenized PHI sentences. Since, the inputs to the deep learning based model are only the PHI sentences (i.e., templatized and tokenized PHI sentences), the processing burden of the at least one second processor **230** is significantly reduced and PHI identification can be performed quickly with less computing resources.

The model may generate two types of vector representations/embeddings- word level representation and character level representation for each word. The at least one second processor **230** may generate word level representations for each word of the one or more templatized PHI sentences using the word embedding layer and may also generate character level representations for each character of the one or more tokenized sentences using a character embedding layer.

The word level representations are vectors of numbers. These vectors capture grammatical function (syntax) and meaning (semantics) of the words, enabling the deep learning model to perform various mathematical operations for PHI identification. Word level representations can only handle seen words i.e., the words which are present in model dictionary. However, there can be a word in the PHI sentences which is not present in model dictionary (known as out-of-vocabulary (OOV) word) so it would be difficult for the model to capture syntax and semantics of such words resulting in inaccurate predictions. To solve such problems, the present disclosure utilizes the character level representations that can handle the OOV words by looking at their character-level compositions. Using the character level representations every single word's vector can be formed even it is OOV word. On the other hand, word embedding can only handle those seen words. Thus, the benefit of character level representations is that it can handle misspelling words, emoticons, new words, and infrequent words. Further, the character level representations are small which helps in reducing model complexity and improving the performance in terms of speed.

In one non-limiting embodiment, the at least one second processor **230** may concatenate the word level representations and the character level representations to generate final representations for each of the one or more templatized PHI sentences. The concatenated final representations may be passed to the sequential representation layer for identifying the one or more PHI in the medical record. The output from the sequential representation layer may be subjected to spatial dropout in 1-dimension to penalize the model for overfitting training data. Final predictions for each word may be obtained at the dense layer. The predictions are corresponding PHI label for the identified PHI.

In an exemplary embodiment, the deep learning based model may be a Bi-LSTM based deep learning model and the sequential representation layer may be a Bi-LSTM layer. However, the present disclosure is not limited thereto and any deep learning model that could understand sequential information could be used in place Bi-LSTM including, but not limited to, LSTM, RNN, GRU, CRF, transformer models (BERT), models with attention, temporal CNN or any combination thereof.

Referring back to **Figure 3**, in one non-limiting embodiment of the present disclosure, the at least one second processor **230** may process the identified PHI sentences using a rule based PHI parser in order to identify any PHI which might be missed out by the deep learning based model (**block 312**- rule based PHI tagging). The usage of rule based PHI parser improves the precision and recall and hence the accuracy of PHI identification. It may be noted here that the rule based parser comprises only few rules e.g., it comprises rules only for the cases on which deep learning based model fails.

Once the PHI has been identified in the input medical record 160, the at least one second processor **230** may perform PHI deidentification by replacing the identified PHI with one or more character strings (**block 314**) to generate anonymized medical record corresponding to the input medical record **160** (**block 316**). The one or more character strings may comprise random character strings or one or more PHI strings equivalent to the identified PHI. The at least one second processor **230** may store a mapping between the identified PHI and corresponding character strings in an encrypted file or hash map (block **318**). The anonymized medical record may be shared with the outside entities (i.e., entities which are outside of a health care facility). The outside entities may include institutions, organizations, or persons.

In one non-limiting embodiment of the present disclosure, the at least one second processor **230** may convert the anonymized medical record back into the original medical record by replacing the character strings with corresponding PHI based on the mapping stored in the encrypted file (block **320**).

In one non-limiting embodiment of the present disclosure, the at least one second processor **230** may redact the PHI in the medical record (e.g., in case of pdf and image file formats of medical records). Thus, the present disclosure provides following functional capabilities: redact, identify, mask, de-identify, and re-identify.
Identify: The PHI is identified in the medical record and tagged with their respective tags in XML format, as shown below:
   **Input:** *"Dr. Jane Doe recommended Drug A to Mr. Alex on 20*/*01*/*22"*
   **Output:** *"<Name> Dr. Jane Doe <*/*Name> recommended Drug A to <Name> Mr. Alex* </*Name*> *on <Date> 20101122 <*/*Date>"*
Mask: The PHI in the medical record is replaced with preset characters/patterns, as shown below:
   **Input:** *"Dr. Jane Doe recommended Drug A to Mr. Alex on 20*/*01*/*22"*
   **Output:** *"XX XXXX XXX recommended Drug A to XX XXXX on XX XX XX*"
De-identify: The PHI in the medical record is replaced with random string pattern and the mapping between the PHI and the random string pattern is stored in the encrypted file, as shown below:
   **Input:** *"Dr. Jane Doe recommended Drug A to Mr. Alex on 20*/*01*/*22"*
   **Output:** *"XA. OKMNIA XYS recommended Drug A to XNX MMN on SDIUY'*
Re-identify: The anonymized medical record is converted back into the original medical record by replacing the random string pattern with corresponding PHI based on the mapping stored in the encrypted file.
   **Input:** *"XA. OKMNIA XYS recommended Drug A to XNX MMN on SDIUY*"
   **Output:** *"Dr. Jane Doe recommended Drug A to Mr. Alex on 20101122"*

Thus, the present disclosure describes efficient techniques data processing techniques for anonymizing medical records by identifying Protected Health Information (PHI) in a medical record using a unique combination of deep learning, smart templatization, and rules. The usage of deep learning models enables the system to be domain and language independent. To reinforce the model predictions, the present disclosure uses the rule based module which predicts any missed out PHI that is identifiable. To achieve a state of the art performance and to reduce the dictionary size of the deep learning models, the present disclosure templatizes certain textual words in the medical record. After identification of the PHI in the medical record, the proposed techniques may mask the identified PHI and store them in a secure database. This de-identified record can be shared with other internal/external entities for further processing or analysis. This proposed techniques also have a capability to re-identify all de-identified PHI words in the medical document. The proposed techniques are independent of medical record type and can handle medical record of any type.

In one non-limiting embodiment of the present disclosure, the proposed techniques may be extended to an automated platform for anonymizing medical records which may be beneficial for health care facilities, outside entities, and researchers. The platform may be provided in the form application programming interface (API) or deployable solutions. The entity willing to anonymize a medical record may upload the medical record and the platform may provide anonymized medical record to entity. This saves additional computational costs and enhances their user experience. The techniques of the present disclosure may utilize a Graphical User Interface (GUI) provided on the computing system **110** so as to enable a convenient and easy processing of medical records (even for non-experts).

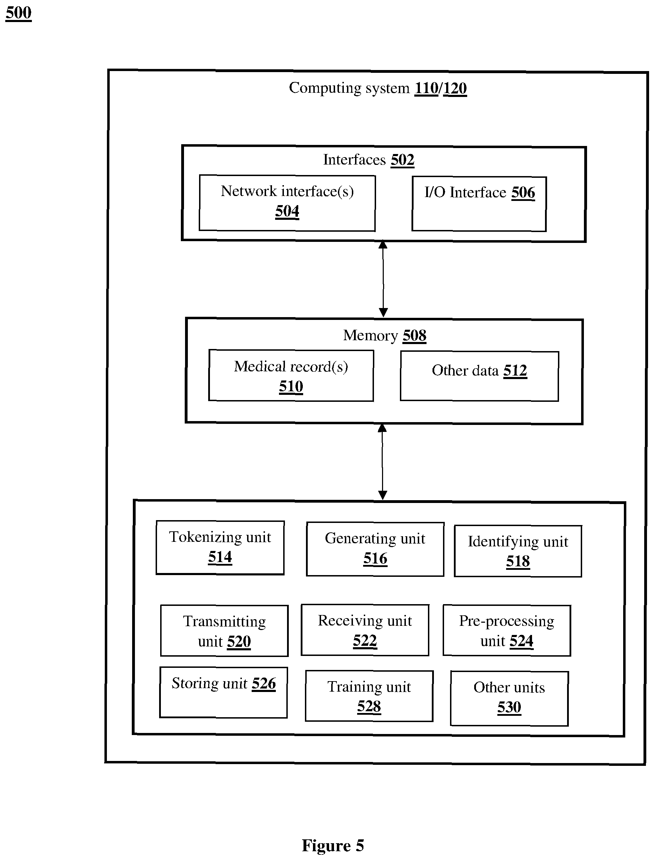

Referring now to **Figure 5****,** which shows a block diagram of a computing system **110, 120,** in accordance with some embodiments of the present disclosure. In one non-limiting embodiment of the present disclosure, the computing systems **110, 120** may comprise various other hardware components such as various interfaces **502,** memory **508,** and various units or means as shown in **Figure 5****.** The units may comprise a tokenizing unit **514,** a generating unit **516,** an identifying unit **518,** a transmitting unit **520,** a receiving unit **522,** a pre-processing unit **524,** a storing unit **526,** a training unit **528,** and various other units **530.** The other units **530** may comprise a display unit, a mapping unit etc. In an embodiment, the units **514-530** may be dedicated hardware units capable of executing one or more instructions stored in the memory **508** for performing various operations of the computing system **110, 120.** In another embodiment, the units **514-530** may be software modules stored in the memory **508** which may be executed by the at least one processor **210, 230** for performing the operations of the computing system **110, 120.**

The interfaces **502** may include a variety of software and hardware interfaces, for example, a web interface, a graphical user interface, an input device-output device (I/O) interface **506,** a network interface **504** and the like. The I/O interfaces **506** may allow the computing system **110, 120** to interact with other computing systems directly or through other devices. The network interface **504** may allow the computing system **110, 120** to interact with one or more data sources **130, 140** either directly or via the network **150.**

The memory **508** may comprise one or more medical records **510,** and other various types of data **512** such as one or more instructions executable by the at least processor **210, 230.** The memory **508** may be any of the memories **240, 260.**

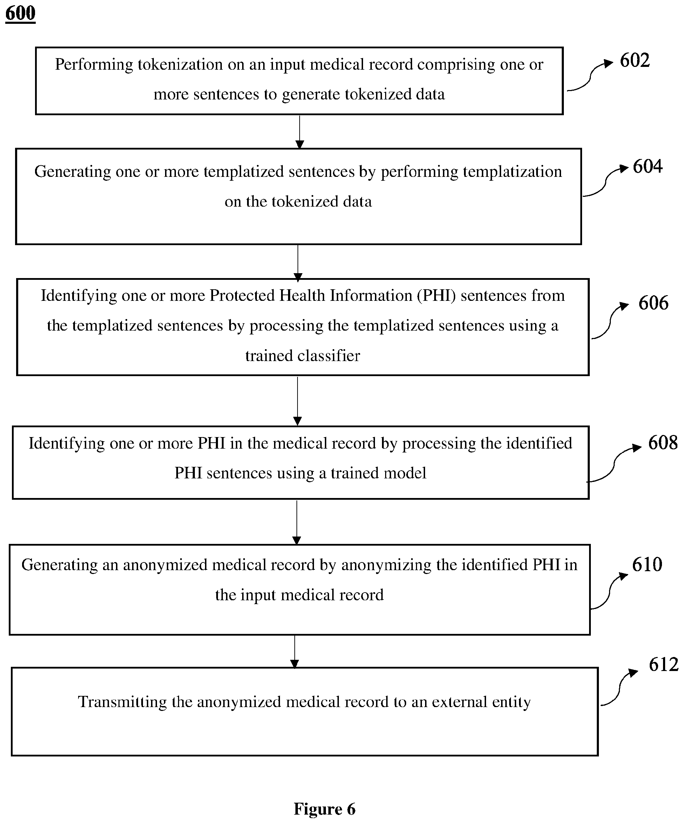

Referring now to **Figure 6****,** a flowchart is described illustrating an exemplary method **600** for anonymizing medical records, according to an embodiment of the present disclosure. The method **600** is merely provided for exemplary purposes, and embodiments are intended to include or otherwise cover any methods or procedures for anonymizing medical records.

The method **600** may include, at block **602,** performing tokenization on an input medical record comprising one or more sentences to generate tokenized data. The tokenized data may comprise one or more tokenized sentences. The operations of block **602** may be performed by the at least one second processor **230** of Figure 2 or by the tokenizing unit **514** of Figure 5.

In one non-limiting embodiment of the present disclosure, the method **600** may include, prior to performing the tokenization, performing pre-processing on the input medical record. The performing the pre-processing may comprise cleaning up the input medical record by performing one or more operations comprising: removing stop words, removing special characters, removing punctuations, stemming, lemmatization, removing extra white spaces, and converting whole medical record in lowercase letters. In one non-limiting embodiment, performing the pre-processing on the input medical record may further comprise merging a sentence of the input medical record with previous and/or next sentences using a deep learning based context merger classifier.

At block **604,** the method **600** may include generating one or more templatized sentences by performing templatization on the tokenized data. Performing the templatization may comprise replacing one or more known patterns in the tokenized data with one or more predefined patterns. The operations of block **604** may be performed by the at least one second processor **230** of Figure 2 or by the generating unit **516** of Figure 5.

At block **606,** the method **600** may include identifying one or more Protected Health Information (PHI) sentences from the templatized sentences by processing the templatized sentences using a trained classifier. Each of the one or more PHI sentences may comprise one or more PHI. The operations of block **606** may be performed by the at least one second processor **230** of Figure 2 or by the identifying unit **518** of Figure 5.

In one non-limiting embodiment of the present disclosure, the trained classifier may be a sequential deep learning classifier. In one non-limiting embodiment of the present disclosure, the operation of block **606** i.e., identifying one or more PHI sentences from the templatized sentences may comprise identifying, using a rule-based classifier, one or more missed out PHI sentences by processing templatized sentences which are classified as non-PHI sentences.

At block **608,** the method **600** may include identifying one or more PHI in the medical record by processing the identified PHI sentences using a trained model. The operations of block **608** may be performed by the at least one second processor **230** of Figure 2 or by the identifying unit **518** of Figure 5.

In one non-limiting embodiment of the present disclosure, the trained model may be an artificial neural network based model, and identifying the one or more PHI in the medical record may comprise generating word level representations for each of the one or more templatized PHI sentences using a word embedding layer of the trained model and generating character level representations for each character of the one or more tokenized sentences using a character embedding layer of the trained model. The method may further comprise concatenating the word level representations and the character level representations to generate final representations for each of the one or more templatized PHI sentences and identifying the one or more PHI in the medical record by processing the final representations using a sequential representation layer of the trained model.

In one non-limiting embodiment of the present disclosure, the method may further comprise identifying one or more missed out PHI in the medical record by processing the one or more PHI sentences using a rule based parser.

At block **610,** the method **600** may include generating an anonymized medical record by anonymizing the identified PHI in the input medical record. The operations of block **610** may be performed by the at least one second processor **230** of Figure 2 or by the generating unit **516** of Figure 5.

In one non-limiting embodiment of the present disclosure, the operation of block **610** i.e., generating an anonymized medical record may comprise generating the anonymized medical record by replacing the identified PHI with one or more character strings, wherein the one or more character strings comprise random character strings or one or more PHI strings equivalent to the identified PHI.

In one non-limiting embodiment of the present disclosure, the method may further comprise storing a mapping between the identified PHI and corresponding character strings in an encrypted file and converting the anonymized medical record back into the original medical record by replacing the character strings with corresponding PHI based on the mapping stored in the encrypted file

At block **612,** the method **600** may include transmitting the anonymized medical record to an external entity. The operations of block **612** may be performed by the at least one second processor **230** of Figure 2 or by the transmitting unit **520** of Figure 5.

The disclosed techniques of anonymizing medical records are time efficient and consume less computing resources compared to the conventional techniques. The disclosed techniques have a higher accuracy compared to other techniques of anonymizing medical records.

The above method **600** may be described in the general context of computer executable instructions. Generally, computer executable instructions can include routines, programs, objects, components, data structures, procedures, modules, and functions, which perform specific functions or implement specific abstract data types.

The order in which the various operations of the methods are described is not intended to be construed as a limitation, and any number of the described method blocks can be combined in any order to implement the method. Additionally, individual blocks may be deleted from the methods without departing from the spirit and scope of the subject matter described herein. Furthermore, the methods can be implemented in any suitable hardware, software, firmware, or combination thereof.

The various operations of methods described above may be performed by any suitable means capable of performing the corresponding functions. The means may include various hardware and/or software component(s) and/or module(s), including, but not limited to the processors **210, 230** of **Figure 2** and the various units of **Figure 5****.** Generally, where there are operations illustrated in Figures, those operations may have corresponding counterpart means-plus-function components. It may be noted here that the subject matter of some or all embodiments described with reference to **Figures 1-5** may be relevant for the method and the same is not repeated for the sake of brevity.

In a non-limiting embodiment of the present disclosure, one or more non-transitory computer-readable media may be utilized for implementing the embodiments consistent with the present disclosure. Certain aspects may comprise a computer program product for performing the operations presented herein. For example, such a computer program product may comprise a computer readable media having instructions stored (and/or encoded) thereon, the instructions being executable by one or more processors to perform the operations described herein. For certain aspects, the computer program product may include packaging material.

Various components, modules, or units are described in this disclosure to emphasize functional aspects of devices configured to perform the disclosed techniques, but do not necessarily require realization by different hardware units. Rather, as described above, various units may be combined in a hardware unit or provided by a collection of interoperative hardware units, including one or more processors as described above, in conjunction with suitable software and/or firmware. The terms "including", "comprising", "having" and variations thereof mean "including but not limited to", unless expressly specified otherwise.

Finally, the language used in the specification has been principally selected for readability and instructional purposes, and it may not have been selected to delineate or circumscribe the inventive subject matter. It is therefore intended that the scope of the invention be limited not by this detailed description, but rather by any claims that issue on an application based here on. Accordingly, the embodiments of the present invention are intended to be illustrative, but not limiting, of the scope of the invention, which is set forth in the appended claims.

## Claims

1. A method of anonymizing medical records, comprising:
performing tokenization on an input medical record comprising one or more sentences to generate tokenized data, wherein the tokenized data comprises one or more tokenized sentences;
generating one or more templatized sentences by performing templatization on the tokenized data, wherein performing the templatization comprises replacing one or more known patterns in the tokenized data with one or more predefined patterns;
identifying one or more Protected Health Information (PHI) sentences from the templatized sentences by processing the templatized sentences using a trained classifier, wherein each of the one or more PHI sentences comprises one or more PHI;
identifying one or more PHI in the medical record by processing the identified PHI sentences using a trained model;
generating an anonymized medical record by anonymizing the identified PHI in the input medical record; and
transmitting the anonymized medical record to an external entity.

2. The method of claim 1, further comprising:
performing pre-processing on the input medical record prior to performing the tokenization, wherein performing the pre-processing comprises cleaning up the input medical record by performing one or more operations comprising:
removing stop words, removing special characters, removing punctuations, stemming, lemmatization, removing extra white spaces, and converting whole medical record in lowercase letters.

3. The method of claim 2, wherein performing pre-processing on the input medical record further comprises:
merging a sentence of the input medical record with previous and/or next sentences using a deep learning based context merger classifier.

4. The method of any preceding claim, wherein identifying one or more PHI sentences from the templatized sentences comprises:
identifying, using a rule-based classifier, one or more missed out PHI sentences by processing templatized sentences which are classified as non-PHI sentences.

5. The method of any preceding claim, wherein the trained classifier is a sequential deep learning classifier, and wherein the method further comprises:
identifying one or more missed out PHI in the medical record by processing the one or more PHI sentences using a rule based parser.

6. The method of any preceding claim, wherein the trained model is an artificial neural network based model, and wherein identifying the one or more PHI in the medical record comprises:
generating word level representations for each of the one or more templatized PHI sentences using a word embedding layer of the trained model;
generating character level representations for each character of the one or more tokenized sentences using a character embedding layer of the trained model;
concatenating the word level representations and the character level representations to generate final representations for each of the one or more templatized PHI sentences; and
identifying the one or more PHI in the medical record by processing the final representations using a sequential representation layer of the trained model.

7. The method of any preceding claim, wherein generating an anonymized medical record comprises:
generating the anonymized medical record by replacing the identified PHI with one or more character strings, wherein the one or more character strings comprise random character strings or one or more PHI strings equivalent to the identified PHI;
storing a mapping between the identified PHI and corresponding character strings in an encrypted file; and
converting the anonymized medical record back into the original medical record by replacing the character strings with corresponding PHI based on the mapping stored in the encrypted file.

8. An apparatus for anonymizing medical records, comprising:
a memory storing computer executable instructions; and
at least one processor in electronic communication with the memory and configured to:
perform tokenization on an input medical record comprising one or more sentences to generate tokenized data, wherein the tokenized data comprises one or more tokenized sentences;
generate one or more templatized sentences by performing templatization on the tokenized data, wherein performing the templatization comprises replacing one or more known patterns in the tokenized data with one or more predefined patterns;
identify one or more Protected Health Information (PHI) sentences from the templatized sentences by processing the templatized sentences using a trained classifier, wherein each of the one or more PHI sentences comprises one or more PHI;
identify one or more PHI in the medical record by processing the identified PHI sentences using a trained model;
generate an anonymized medical record by anonymizing the identified PHI in the input medical record; and
transmit the anonymized medical record to an external entity.

9. The apparatus of claim 8, wherein the at least one processor is further configured to:
perform pre-processing on the input medical record prior to performing the tokenization, wherein performing the pre-processing comprises cleaning up the input medical record by performing one or more operations comprising:
removing stop words, removing special characters, removing punctuations, stemming, lemmatization, removing extra white spaces, and converting whole medical record in lowercase letters.

10. The apparatus of claim 9, wherein to perform pre-processing on the input medical record, the at least one processor is further configured to:
merge a sentence of the input medical record with previous and/or next sentences using a deep learning based context merger classifier.

11. The apparatus of any of claims 8-10, wherein the at least one processor is further configured to:
identify, using a rule-based classifier, one or more missed out PHI sentences by processing templatized sentences which are classified as non-PHI sentences.

12. The apparatus of any of claims 8-11, wherein the trained classifier is a sequential deep learning classifier and wherein the at least one processor is further configured to:
identify one or more missed out PHI in the medical record by processing the one or more PHI sentences using a rule based parser.

13. The apparatus of any of claims 8-12, wherein the trained model is an artificial neural network based model, and wherein to identify the one or more PHI in the medical record, the at least one processor is further configured to:
generate word level representations for each of the one or more templatized PHI sentences using a word embedding layer of the trained model;
generate character level representations for each character of the one or more tokenized sentences using a character embedding layer of the trained model;
concatenate the word level representations and the character level representations to generate final representations for each of the one or more templatized PHI sentences; and
identify the one or more PHI in the medical record by processing the final representations using a Bi-directional Long Short Term Memory (Bi-LSTM) layer of the trained model.

14. The apparatus of any of claims 8-13, wherein to generate the anonymized medical record, the at least one processor is further configured to:
generate the anonymized medical record by replacing the identified PHI with one or more character strings, wherein the one or more character strings comprise random character strings or one or more PHI strings equivalent to the identified PHI, and
wherein the at least one processor is further configured to:
store a mapping between the identified PHI and corresponding character strings in an encrypted file; and
convert the anonymized medical record back into the original medical record by replacing the character strings with corresponding PHI based on the mapping stored in the encrypted file.

15. A non-transitory computer readable media storing one or more instructions executable by at least one processor, the one or more instructions comprising:
one or more instructions for performing tokenization on an input medical record comprising one or more sentences to generate tokenized data, wherein the tokenized data comprises one or more tokenized sentences;
one or more instructions for generating one or more templatized sentences by performing templatization on the tokenized data, wherein performing the templatization comprises replacing one or more known patterns in the tokenized data with one or more predefined patterns;
one or more instructions for identifying one or more Protected Health Information (PHI) sentences from the templatized sentences by processing the templatized sentences using a trained classifier, wherein each of the one or more PHI sentences comprises one or more PHI;
one or more instructions for identifying one or more PHI in the medical record by processing the identified PHI sentences using a trained model;
one or more instructions for generating an anonymized medical record by anonymizing the identified PHI in the input medical record; and
one or more instructions for transmitting the anonymized medical record to an external entity.
